# EUROPEAN PATENT SPECIFICATION

(11) **EP 1 737 569 B1**
(45) Date of publication and mention of the grant of the patent: **26.12.2007**
(21) Application number: 04703950.8
(22) Date of filing: 21.01.2004
(51) Int. Cl.: B01J 23/72, B01J 37/08, C07C 29/154, B01J 23/745, B01J 23/89

(54) **CHROMIUM-FREE CATALYSTS OF METALLIC CU AND AT LEAST ONE SECOND METAL**
CHROMFREIE KUPFERMETALL-KATALYSATOR MIT WENIGSTENS EINEM WEITEREN METALL
CATALYSEURS EXEMPT DE CHROME, A BASE DE CU METALLIQUE ET D'AU MOINS UN SECOND METAL

(43) Date of publication of application: 03.01.2007
(73) Proprietor: Avantium International B.V., 1014 BV Amsterdam (NL); Universiti Malaya, Kuala Lumpur 50603 (MY)
(72) Inventor: SIJPKES, André, Harmen, NL-1338 SZ Almere (NL); VAN DER PUIL, Nelleke, NL-1097 CL Amsterdam (NL); VAN DEN BRINK, Peter, John, NL-3972 EJ Driebergen-Rijsenburg (NL); DE KEIJZER, Adrianus,H.J.F., Mgr.Nolenstraat 23, BN Hoogereheide (NL); ABDUL HAMID, Sharifah, Bee, 13 Jalan Cecawi, 47810 Petaling Jaya (MY)
(74) Representative: de Lang, Robbert-Jan
(86) International application number: PCT/NL2004/000051
(87) International publication number: WO 2005/070537

(56) References cited:
- EP-A- 0 320 074
- EP-A- 0 372 544
- GB-A- 1 600 517
- US-A- 4 279 781
- US-A- 4 291 126
- US-A- 4 552 861
- US-A- 4 876 402
- US-A- 5 302 568
- WANG Z ET AL: "Studies on the active species and on dispersion of Cu in Cu/SiO2 and Cu/Zn/SiO2 for hydrogen production via methanol partial oxidation" INTERNATIONAL JOURNAL OF HYDROGEN ENERGY, ELSEVIER SCIENCE PUBLISHERS B.V., BARKING, GB, vol. 28, no. 2, February 2002 (2002-02), pages 151-158, XP004394393 ISSN: 0360-3199

## Description

The present invention relates to a method for the preparation of a chromium-free catalyst comprising Cu and at least one second metal in metallic or oxidic form, a catalyst obtainable by such method and the use of said catalyst for the hydrogenation of fatty acids and fatty esters to fatty alcohols and other esters or di-esters to their corresponding alcohols.

Copper containing catalysts are well known catalysts for the hydrogenation/hydrogenolysis of fatty acids and fatty esters to fatty alcohols. Fatty alcohols are used as intermediates for the production of surfactants, soaps and base oils, and additives for lubricants. Palm oil and palm kernel oil, for example, are commonly used as starting materials for the production of C₁₂-C₁₈ fatty alcohols.

However, severe conditions are especially required for the production of higher aliphatic alcohols. In industrially available processes, hydrogenation is carried out at temperatures of 200-300 °C, pressures of 200-300 bar and high H₂/substrate ratios usually in the presence of a copper chromium catalyst.

Cu-Cr catalysts are currently the commercially most successful catalysts employed for this process. These catalysts have adequate hydrogenation activity and adequate resistance to the fatty acids in the reaction mixture. However, these catalysts have one major drawback: like all catalysts they lose their activity with time, and as chromium compounds are toxic, they need to be handled prudently, and a great deal of labour and cost is spent in treating/recovering the waste catalyst. In addition, since many palm oil derived chemical intermediates have a final application in household products (soaps, detergents, cosmetics, etc.), chromium contamination of the product stream would have to be monitored.

In the art, there is a need for chromium-free Cu catalysts for the hydrogenation/hydrogenolysis of fatty acids and fatty esters, which are not toxic, and which are capable of performing under severe conditions, i.e. high temperatures, pressures and/or H₂/substrate ratios, or, alternatively, are capable of performing the hydrogenation/hydrogenolysis with comparable conversion, selectivity, and yields under milder circumstances.

Several chromium-free Cu catalysts have been developed in the art, e.g. Cu-Zn catalysts (see e.g. US 5,475,159 and US 5,157,168), Cu-Fe catalysts (see e.g. US 4,278,567 and US 5,763,353) and catalysts containing only Cu as active metal (see e.g. US 5,403,962 and WO 97/34694). Generally, these chromium-free Cu catalysts have been prepared by co-precipitation of the catalyst metal components, i.e. preparation of a solution containing the metal salts, optionally combined with a solution of an inert carrier metal precursor such as e.g. Al salts, or with inert carrier metal oxides of Al or Si, and reaction of the resultant solution or slurry with an alkaline aqueous solution to obtain a precipitate of a mixture of metal hydroxides or oxides, after which the precipitate is washed and dried, followed by calcination.

Accordingly, these chromium-free Cu catalysts have the advantage that they do not comprise toxic Cr substances. Generally, however, the chromium-free Cu catalysts obtained in the art thus far suffer in activity or selectivity in comparison with Cu-Cr catalysts, their acid resistance is low, or they are not able to withstand the harsh hydrogenation reaction conditions. It is thought that the co-precipitation has the drawback that separate metals precipitate at different pH values such that at least part of the metals will not have intermixed at an atomic level, to result in the formation of distinct metal clusters at the catalyst surface.

An alternative preparation method for chromium-free Cu catalysts is disclosed in US 5,759,947. Said method comprises the preparation of a solution containing the metal salts as above, whereto the complexing agent citric acid is added, followed by impregnation of spherical support therewith.

It has now surprisingly been found that upon impregnation with a solution comprising ions of a complexing agent, said solution having a pH above 5, catalysts were obtained having an improved activity or selectivity, or relatively high activity, selectivity, and yield at low temperatures and pressures in comparison with the catalysts known in the art.

Therefore, it was an object of the present invention to prepare novel chromium-free Cu catalysts that had improved activity or selectivity, or preferably combinations thereof. It was also an object of the present invention to prepare such catalysts that were capable of catalysing the hydrogenation under milder conditions in comparison with conventional Cu-Cr catalysts.

Thus, the invention relates to a novel method for the preparation of a chromium-free catalyst comprising Cu and at least one second metal in metallic or oxidic form, comprising the steps of:
a) preparing a final solution comprising ions of Cu and the at least one second metal, said final solution additionally comprising ions of an organic complexing agent and having a pH of above 5;
b) contacting said final solution with inert carrier to form a final solution/carrier combination;
c) optionally, drying the final solution/carrier combination;
d) calcining the final solution/carrier combination obtained in step c) or d) to yield Cu and the at least one second metal in oxidic form; and
e) reducing at least part of the thus obtained oxidic Cu on the carrier.

It was found that the chromium-free Cu catalysts thus obtained showed promising activity and selectivity, particularly in the hydrogenation of fatty esters and fatty acids.

Without wishing to be bound by theory, it is thought that the pH of the final solution is important for keeping all metals present in a uniform solution such that the metals are fully intermixed at an atomic level and no distinct metal clusters are formed at the catalyst surface.

As used herein, with the term "at least one second metal" is meant that in addition to Cu at least one second metal is provided in the catalyst; however, it is also possible that the catalyst comprises two, three, four, etc. different metals in addition to the Cu. Preferably, the at least one second metal is chosen from group IB, group IIB, and group VIII metals and may comprise Zn, Fe, Ni and Co. The at least one second metal is preferably chosen from Fe and Zn.

In step a), a final solution is prepared comprising ions of Cu and of the at least one second metal, said final solution additionally comprising ions of a complexing agent and having a pH of above 5.

The ions of the complexing agent, herein also referred to as "complexing ions", may be ions derived from any organic complexing agent, such as citrate ions, lactate ions, EDTA, etc. It is however preferred that said complexing ions are citrate ions, provided e.g. as citric acid or in the form of a salt.

Said final solution may be prepared by dissolution of one or more Cu-salts and of one or more salts of the at least one second metal in a single container, followed by the addition of the complexing agent, e.g. in the form of citric acid, to the said container and optionally, if required, adjustment of the pH to a pH of above 5.

It is also possible that both the metal ions and the ions of the complexing agent are provided in the solution as a single salt. E.g. Cu citrate can be used to provide both for the required Cu ions as well as for the required citrate ions. Accordingly, the second metal can also be provided as e.g. a citrate salt. In this respect it is to be noted that in addition to such a salt comprising both the metal and the complexing agent, the metal ions and/or the ions of the complexing agent can additionally be provided, if necessary, by the addition of additional other metal salts, or as citric acid, respectively.

Alternatively, said final solution may be prepared by combining separate metal salt solutions, such as e.g. a solution of one or more Cu-salt, e.g. Cu-nitrate, and a solution of one or more of a salt of the at least one second metal, e.g. Fe-nitrate. The separate metal salt solutions may comprise more than one metal. In case of the presence of more than one second metal, ions of the second metal may be provided in separate solutions are in a combined solution of the at least one second metals.

The pH of the final solution is above 5. The pH may be adjusted by the addition of any base, such as e.g. NH₄OH NaOH, KOH and Ca (OH)₂, or by the dissolution of the metal salts in any suitable base. Preferably, NH₄OH is used to adjust the pH since in contrast to some of the metal bases it is not harmful to the catalyst and will therefore not have to be removed.

In case citrate salts of the required metal ions are used to prepare the final solution, the said salts are preferably dissolved in concentrated ammonia.

In step b), the final solution is contacted with inert carrier to form a final solution/carrier combination. Contacting of the final solution with the inert carrier may take place by contacting the inert carrier in the form of a porous, dry powder with the final solution, or by mixing the final solution with e.g. the inert carrier in liquid form, such as in a slurry or sol. Alternatively, the carrier may be provided in the form of porous, shaped particles, such as extrudates, pellets, spheres, or any other shape.

The inert carrier may be any conventional carrier, such as e.g. diatomaceous earth, alumina, silica gel, magnesia, silica-magnesia, calcia, zirconia, titania, zeolite, and silica-alumina. The carrier may be provided in the form of a dry powder or in the form of an (aqueous) colloidal suspension, also called slurry, such as e.g. silica sol. The carrier can be provided as a mixture of different powders, or as slurry, optionally comprising different porous powders or porous shaped particles and colloidal suspensions.

Optionally, the contacting step b) is followed by a drying step c), said drying step preferably being carried out at a temperature in the range of 80 to 140 °C. Drying of the final solution/carrier combination can be conducted by any conventional drying method known in the art, such as e.g. amorphous drying, spray-drying, etc. These drying methods are well known and highly suitable in an industrial environment. Upon drying the final solution/carrier combination the metals will precipitate to form mixed metal species on a microscopic, atomic level. In this way, catalysts are prepared comprising a variety of metal species mixed on an atomic level in a range of atomic ratios.

Subsequently, in step d), the final solution/carrier combination is calcined in air to burn off the organic and inorganic residues from the precursor salts, and to convert the metal precursors to their respective metal oxides and mixed metal oxides. The calcination step may also immediately be employed on the final solution/carrier combination of step b), thereby omitting step c), as drying will then take place early during the calcination. However, an intermediate drying step c) is preferred. Calcination is preferably performed at a temperature in the range of 300-900 °C, more preferably of 400-600 °C, most preferably of 400-500 °C, preferably under an atmosphere in which oxygen is present to yield a Cu catalyst precursor. The catalysts thus obtained have improved activity or selectivity or a combination thereof.

In the hydrogenation reactions wherein the catalyst is finally employed, the catalyst is used in the at least partially reduced form, i.e. comprising at least part of the Cu and the at least one second metal in metallic form. The (partial) reduction of Cu is well known in the art and as such, any skilled practitioner will be capable of reducing the Cu catalyst precursor. Any methods for reduction of the Cu catalyst precursor may be employed, which include e.g. any method of gas phase reduction and liquid phase reduction carried out in a solvent such as e.g. hydrocarbons, including liquid paraffin, dioxane, aliphatic alcohols and fatty esters. For example, in case the reduction is carried out in hydrogen gas, it is preferably carried out until formation of water is not observed or absorption of hydrogen is not observed. Alternative reducing agents comprise carbon monoxide, ammonia, hydrazine, formaldehyde, ethylene and lower alcohols such as methanol. When reduction is carried out in a solvent in the presence of hydrogen gas, it is preferably carried out until absorption of hydrogen gas is not observed at temperatures of 150-350 °C. The reduction step e) may also be conducted *in situ* in the hydrogenation reactor.

It is possible that also one or more of the at least one second metal present are (partially) reduced; however, for catalytic action it is mostly sufficient that the Cu is at least partially reduced. For example, in case of a Cu-Fe catalyst on silica it is known that Fe may form a ferrosilicate which cannot be reduced to metallic species. Thus, after reduction the catalytic species may be a (partly) reduced Cu, optionally in combination with (partly) reduced Fe on a ferrosilicate support.

In a preferred embodiment, step a) comprises the step of preparing said final solution by combining at least a first solution comprising ions of Cu with at least a second solution comprising ions of at least one second metal. Thus, the pH of the solutions can be controlled separately and precipitation of the metals can be avoided. It is preferred that the said first and second solutions are compatible. With "compatible" as herein used, it is meant that no precipitation of separate metals occurs upon combining of the first and second solutions. Said first solution may be prepared from any Cu salt, and a complexing agent, such as e.g. citric acid, and adjustment of the pH to above 5, or may alternatively be prepared by dissolution of the salt of the Cu and the complexing agent, preferably in a basic solution such as ammonia, and, if required, adjustment of the pH to above 5. Similarly, said second solution may be prepared from any salt of the at least one second metal, followed by the addition of complexing agent, such as e.g. citric acid, and adjustment of the pH to above 5, or may alternatively be prepared by dissolution of the salt of the at least one second metal and the complexing agent, preferably in a basic solution such as ammonia, and, if required, adjustment of the pH to above 5. Any first solution, regardless of the preparation method thereof, can be combined with any second solution, regardless of the preparation method thereof, to obtain the final solution, as long as the first and second solution are compatible. In case metal citrate is used to prepare the solutions, it is preferred that these are dissolved in ammonia.

Preferably, the first solution and the second solution both comprise ions of the complexing agent in a similar concentration, and are thus in this regard compatible. With "a similar concentration" as herein used, it is meant that the concentration differs at most by a factor 2, preferably at most by a factor 1.6, more preferably at most by a factor 1.3.

Moreover, it is preferred that both the first and the second solution have a pH above 5, such that precipitation of the Cu or the at least one second metal due to pH differences can be avoided. It is most preferred that the first and second solution have a similar pH. With "a similar pH" as herein used, it is meant that the pH difference between the first and second solution is at most 1.5, preferably at most 1.0, more preferably at most 0.5.

In one embodiment, said chromium-free catalyst further comprises at least one third metal, said third metal being chosen from Pd, Pt, Ru and Rh. The at least one third metal can be considered as promoter metal.

Said third metal can be added to the final solution, or to the above first and second solutions. Further, a third metal can be provided in a third solution, that preferably is compatible with the above first and second solution, preferably both with regard to pH and concentration of ions of the complexing agent. Thus, it is preferred that the third solution comprises ions of the complexing agent in a similar concentration as the first and the second solution. Moreover, it is preferred that the third solution has a pH of above 5, and preferably a pH that is similar to the pH of the first and second solutions.

However, it is also possible that the calcined final solution/carrier combination of step d) is impregnated with a solution comprising the at least one third metal, followed by another round of calcination. This is particularly suitable for the incorporation of the noble metal promoters.

It is preferred that the pH of the final solution is above 6, as it was found that under these circumstances best catalysts were obtained. In case the final solution is prepared from a first and second and optionally a third solution, it is preferred that the pH of the first, second and third solution is above 6.

It is preferred that the concentration of Cu ions in the final solution is in the range of 0.001-0.3 g/mL, more preferably of 0.005-0.15 g/mL. Preferably, the amount of Cu ions in the final solution is such that a catalyst is obtained comprising 1-50 %wt, more preferably 10-30 %wt, and most preferably 15-25 %wt Cu. It was found that excellent catalysts were obtained using such amounts of Cu.

The concentration of ions of the complexing agent in the final solution preferably is in the range of 0.001-1.5 g/mL, more preferably of 0.15-0.5 g/mL. Most preferably, the amount of ions of the complexing agent in the final solution is such that the molar ratio of metal to complexing agent is in the range of 0.1-5, more preferably 0.5-2, and most preferably 0.75-1.25, as it was found that the best catalysts were obtained with such molar ratios, particularly with molar ratios around 1.

In one embodiment, the concentration of the at least one second metal in the final solution is in the range of 0.001-0.3 g/mL, preferably in the range of 0.005-0.15 g/mL. Preferably, the amount of the at least one second metal in the final solution is such that catalyst is obtained with an atomic ratio of Cu to the at least one second metal in the range of 0.01-10, more preferably in the range of 0.1-5, and most preferably in the range of 0.3-3.0.

In one further embodiment, the concentration of ions of the at least one third metal in the final solution is in the range of 0.0001-0.03 g/mL, preferably in the range of 0.0005-0.015 g/mL. Preferably, the amount of the at least one third metal is such that catalyst is obtained with an atomic ratio of the at least one third metal to Cu is in the range of 0.001-0.05, and more preferably in the range of 0.001-0.01.

In a further embodiment, the method according to the present invention comprises an additional step g) of pulverising the obtained catalyst. Said pulverising may be important as the catalysts may be used in a liquid phase batch reactor, which requires the catalyst to be in the form of a fine powder. In this case, the preferred particle size is in the range of 0.1-250 µm, more preferably in the range of 1-100 µm, and most preferably in the range of 5-25 µm. Another advantage of pulverising the obtained catalyst is that the catalytic material is homogenised during the pulverisation of the catalyst material. It should be understood that the thus obtained catalyst material can be further treated by shaping the obtained fine powder to pellets, by extrusion, or by any other means of shaping to larger catalyst bodies known in the art. The object of such shaping is to render the catalyst suitable for testing in other types of reactors, such as fixed bed reactors, or any other types of reactors known in the art.

In a preferred embodiment, the at least one second metal is chosen from one or more of Fe, Zn, Co, Ni, or a combination thereof. It was for example found that certain Cu-Fe catalysts obtained according to the present invention were capable of catalysing the hydrogenation reaction at lower pressures in comparison with conventional catalysts. Moreover, many of the Cu-Fe and Cu-Zn catalysts according to the present invention performed better than conventionally prepared catalysts with regard to activity, selectivity or a combination thereof.

In a further preferred embodiment the at least one third metal is chosen from one or more of Pd, Ru, Pt, Rh, or a combination of two or more thereof.

It is preferred that the inert carrier is chosen from alumina, silica, silica-alumina, titania, magnesia, zirconia, zinc oxide, or any combination thereof, as use of these carriers in the preparation of the catalyst according to the present invention resulted in particularly good catalysts. It is more preferred that the inert carrier is chosen from silica, magnesia and zirconia, as it was found that best results were thus obtained.

Preferably, the inert carrier is present in an amount of 50-95 %wt, preferably 50-90 %wt, most preferably 70-85 %wt, as the thus obtained catalyst is highly stable and displays a high activity. Moreover, a relatively high concentration of cheap carrier material is advantageous from an economic point of view.

In a second aspect, the present invention relates to a chromium-free catalyst comprising Cu and at least one second metal obtainable by any method of the present invention. Such Cu catalyst shows improved activity, selectivity or a combination thereof over conventional Cu catalysts. Alternatively, said catalyst may allow for milder hydrogenation reaction conditions.

It is preferred that said catalyst comprises at least 5 %wt Cu and has an atomic ratio of Cu to the at least one second metal of 0.1-10.

Also, the present invention relates to a chromium-free Cu-Zn catalyst supported on silica, zirconia or magnesia, comprising 5-50 %wt, preferably 10-30 %wt (Cu + Zn) and having a Cu to Zn ratio of 0.1-10 at/at, preferably 0.5-5 at/at, more preferably 1-4 at/at. It was now found for the first time that Cu-Zn catalysts supported on silica, zirconia or magnesia as inert carriers performed much better with regard to activity and/or selectivity in comparison with known Cu-Zn catalysts.

It is preferred that said chromium-free Cu-Zn catalyst further comprises as at least one second metal Co or Ni, or a combination thereof. The incorporation of Co or Ni in the chromium-free Cu-Zn catalyst according to the invention was shown to be advantageous for activity and/or selectivity of the said catalyst.

In a further embodiment, said chromium-free Cu-Zn catalyst further comprises at least one third metal chosen from Rh, Ru, Pd and Pt, or combinations of two or more thereof. It was found that the addition of the at least one third metal often improved activity and/or selectivity of the chromium-free Cu-Zn catalysts.

Best results were obtained with a chromium-free Cu-Zn catalyst as described above having a ratio of (Cu + Zn) to the at least one third metal of 0.0001-0.5 at/at, preferably of 0.001-0.01 at/at.

In yet another aspect, the present invention relates to a chromium-free Cu-Fe catalyst supported on silica, zirconia, or magnesia, comprising 5-50 %wt, preferably 10-30 %wt (Cu + Fe) and having a Cu to Fe ratio of 0.1-10 at/at, preferably 0.5-5 at/at, more preferably 1-4 at/at. It was now found for the first time that Cu-Fe catalysts supported on silica, zirconia or magnesia as inert carriers performed much better with regard to activity and/or selectivity in comparison with known Cu-Fe catalysts.

Preferably, said chromium-free Cu-Fe catalyst according to the present invention further comprises as at least one second metal Co or Ni, or a combination thereof, as addition of these metals further improved activity and/or selectivity.

In a further embodiment, said chromium-free Cu-Fe catalyst according to the present invention further comprises at least one third metal chosen from Rh, Ru, Pd and Pt, or a combination of two or more thereof. It was found that the addition of the at least one third metal, said third metal being a promoter metal, generally improved activity and/or selectivity of the chromium-free Cu-Fe catalysts.

Preferably, the chromium-free Cu-Fe catalyst has a ratio of (Cu + Fe) to the at least one third metal of 0.0001-0.5 at/at, preferably of 0.001-0.01 at/at, as thus best results were obtained for activity and/or selectivity.

In a further aspect the present invention relates to the use of a chromium-free catalyst according to the present invention for the hydrogenation of fatty acids, fatty esters, esters and diesters to fatty alcohols, alcohols and dialcohols, respectively. Non-limiting examples of such fatty acids and fatty esters include linear or branched, saturated or unsaturated fatty acid having one or more carbons, esters of alcohols with the above fatty acids, such as e.g. caproic acid, caprylic acid, capric acid, lauric acid, myristic acid, palmitic acid, stearic acid, isostearic acid, oleic acid, adipic acid, and sebacic acid. Non-limiting examples of fatty esters include caproic ester, caprylic ester, capric ester, lauric ester, myristic ester, palmitic ester, stearic ester, isostearic ester, oleic ester, adipic ester, and sebacic ester.

The fatty acids or fatty esters described above may be hydrogenated using any reaction method, such as e.g. a suspension reaction method, a fixed bed reaction method or a fluidised bed reaction method. A solvent can be used for the reaction but in light of productivity the reaction is preferably carried out in the absence of a solvent. If a solvent is used, a solvent, which does not exert an adverse effect on the reaction such as alcohol, dioxane and hydrocarbon, is selected. The reaction temperatures are generally in the range of 100-300 °C; the reaction pressures are generally in the range of 100-300 bar.

### Examples

The present invention will now be further illustrated in the following examples, which are in no way meant to limit the scope of the present invention.

### Example 1 Preparation of a Cu-Fe on silica catalyst

A first solution was prepared by dissolving 50.0 g copper nitrate trihydrate (Sigma-Aldrich) and 43.5 g citric acid monohydrate (Sigma-Aldrich) in about 150 g water. The solution had a pH of about 0.5, which was increased to pH 7 by adding small amounts of 30% ammonia (Sigma-Aldrich). When the solution had reached pH 7, water was added to obtain 250 mL solution. A second solution was prepared in a similar fashion using 50.0 g iron nitrate nonahydrate (Sigma-Aldrich) and 26.0 g citric acid monohydrate. To a sample of 1 g silica (Aerosil 300, Degussa) was added 3.97 mL of the Cu solution and 2.21 mL of the Fe solution, after which the slurry was stirred for five minutes. The slurry was then dried at 120 °C for 12 h and calcined at 450 °C for 2 h. Finally, the catalyst precursor was crushed to a powder. The catalyst composition was 20 g Cu-Fe per 100 g catalyst with a Cu to Fe at/at ratio of 3:1.

### Comparative example 1A Preparation of a Cu-Fe on silica catalyst

A first solution was prepared by dissolving 50.0 g copper nitrate trihydrate in water to obtain 250 mL solution. A second solution was prepared by dissolving 50.0 g iron nitrate nonahydrate in water to obtain 250 mL solution. Aliquots of 3.97 mL of the Cu solution and 2.21 mL of the second solution were mixed and diluted with water to obtain 25 mL of a third solution. To this solution was added 1 g of silica powder (Aerosil 300). While stirring, an ammonia solution (2 mol/L) was added to the thus obtained slurry at a rate of 0.2 mL/min. After reaching a pH of 9, a precipitate had formed, which was washed with water. After drying (2 h at 120 °C) and calcination (2 h at 450 °C) a catalyst precursor was obtained with a composition of 20 g Cu-Fe per 100 g catalyst with a Cu to Fe at/at ratio of 3:1.

### Comparative example 1B Preparation of a Cu-Fe on silica catalyst

A catalyst was prepared in the same manner as described in Comparative Example 1A, except that a 0.2 M ammonia solution was used for the precipitation reaction.

### Comparative example 2 Preparation of a Cu-Cr-Ba-Mn-Si catalyst (Example 1 of US 5,124,491, Henkel, 1992)

A first solution was prepared by dissolving 48.25 g CrO₃ in 265 g water, which was heated to 60 °C. Next, 97 g 28-30% ammonia was added. The addition of ammonia changed the colour of the solution from orange to light orange-brown, and the pH increased from <1 to 7.9. A second solution was prepared by dissolving 2.50 g Ba-nitrate, 102.84 g Cu-nitrate, and 8.67 g Mn-nitrate in 265 g water. A clear blue solution was obtained, which was heated to 60 °C. To this solution was added 1.83 g of a 40% silica sol (Ludox AS-40, Sigma-Aldrich). The second solution was added to the Cr solution through a funnel in about 20 minutes, upon which the Cr solution turned dark green. After adding all of the solution, the Cr-Cu-Mn-Ba solution remained dark green, and the pH was 7. After cooling the solution, a brown precipitate had formed. The solution + precipitate was poured over a filter (glass, P2) to separate the dark green solution from the precipitate. The precipitate was washed six times with 250 mL water. After the last washing, the precipitate was transferred to a dish, dried and calcined (heat to 105 °C (2 °C/min), heat at 105 °C (12 h), heat to 500 °C (2 °C/min), heat at 500 °C (2 h), cool). A dark brown catalyst precursor was obtained with a composition of 38.5% Cu, 28.3% Cr, 4.6% Ba, 3.5% Mn, 0.8% Si.

### Example 2A Preparation of a Cu-Zn on magnesia catalyst

A first solution was prepared by dissolving 49.1 g copper citrate Pfaltz and Bauer) in 104.2 g 30% ammonia. A second solution was prepared by dissolving 50.4 g zinc citrate dihydrate (Sigma-Aldrich) in 102.8 g 30% ammonia. To a sample of 1 g magnesia (E-10, DSP) was added 1.00 mL of the Cu solution and 1.87 mL of the Zn solution, after which the slurry was stirred for five minutes. The slurry was then dried at 120 °C for 12 h and calcined at 450 °C for 2 h. Finally, the catalyst precursor was crushed to a powder. The catalyst composition was 20 g Cu-Zn per 100 g catalyst with a Cu to Zn at/at ratio of 1:1.

### Example 2B Preparation of a Cu-Zn-Co on magnesia catalyst

Copper and zinc citrate solutions were prepared as in Example 2A. A third solution was prepared by dissolving 10.5 g cobalt citrate (STREM) in 104.1 g 30% ammonia. To a sample of 1 g magnesia (E-10, DSP) was added 1.51 mL of the Cu solution, 0.63 mL of the Zn solution, and 0.135 mL of the Co solution, after which the slurry was stirred for five minutes. The slurry was then dried at 120 °C for 12 h and calcined at 450 °C for 2 h. Finally, the catalyst precursor was crushed to a powder. The catalyst composition was 20 g Cu-Zn-Co per 100 g catalyst with a Cu to Zn to Co at/at ratio of 3:1:0.04.

### Example 2C Preparation of a Cu-Zn-Co on magnesia catalyst

Solutions of copper, zinc, and cobalt nitrates, with equimolar amounts of citric acid, and their pH adjusted to pH 7 with 30% ammonia, were prepared as described in Example 1. To a sample of 1 g magnesia (E-10, DSP) was added 3.75 mL of the Cu solution, 1.52 mL of the Zn solution, and 0.121 mL of the Co solution, after which the slurry was stirred for five minutes. The slurry was then dried at 120 °C for 12 h and calcined at 450 °C for 2 h. Finally, the catalyst precursor was crushed to a powder. The catalyst composition was 20 g Cu-Zn-Co per 100 g catalyst with a Cu to Zn to Co at/at ratio of 3:1:0.04.

### Example 3 Preparation of a Cu-Zn on zirconia catalyst

Copper citrate and zinc citrate solutions were prepared as in Example 2. To a sample of 1 g zirconia powder (NNC100, Daiichi) was added 1.52 mL of the Cu solution, 0.94 mL of the Zn solution, after which the slurry was stirred for five minutes. The slurry was then dried at 120 °C for 12 h and calcined at 450 °C for 2 h. Finally, the catalyst precursor was crushed to a powder. The catalyst composition was 20 g Cu-Zn per 100 g catalyst with a Cu to Zn ratio of 3:1.

### Example 4 Preparation of a Cu-Fe-Co on titania catalyst

A copper citrate solution was prepared as in Example 2. A second solution was prepared by dissolving 50.3 g iron citrate dihydrate (Sigma-Aldrich) in 102.3 g 30% ammonia. A third solution was prepared by dissolving 10.5 g cobalt citrate (STREM) in 104.1 g 30% ammonia. To a sample of 1 g titania powder (P25, Degussa) was added 1.51 mL of the Cu solution, 0.94 mL of the Zn solution, and 0.142 mL of the Co solution, after which the slurry was stirred for five minutes. The slurry was then dried at 120 °C for 12 h and calcined at 450 °C for 2 h. Finally, the catalyst precursor was crushed to a powder. The catalyst composition was 20 g Cu-Fe-Co per 100 g catalyst with a Cu to Fe to Co at/at ratio of 3:1:0.4.

### Example 5A Preparation of a Cu-Zn-Ni on magnesia catalyst

Copper citrate and zinc citrate solutions were prepared as in Example 2. A third solution was prepared by dissolving 10.1 g nickel citrate (Alfa Aesar) in 108.1 g 30% ammonia. To a sample of 1 g magnesia (E-10, DSP) was added 1.51 mL of the Cu solution, 0.63 mL of the Zn solution, and 0.11 mL of the Ni solution, after which the slurry was stirred for five minutes. The slurry was then dried at 120 °C for 12 h and calcined at 450 °C for 2 h. Finally, the catalyst precursor was crushed to a powder. The catalyst composition was 20 g Cu-Zn-Ni per 100 g catalyst with a Cu to Zn to Ni at/at ratio of 3:1:0.4.

### Example 5B Preparation of a Cu-Zn-Ni on zirconia catalyst

A catalyst was prepared analogous to Example 5A, except that zirconia was used as a carrier. The catalyst composition was 20 g Cu-Zn-Ni per 100 g catalyst with a Cu to Zn to Ni at/at ratio of 3:1:0.4.

### Example 6 Preparation of a Cu-Fe-Ni on silica catalyst

Solutions of copper, iron, and nickel nitrates, with equimolar amounts of citric acid, and their pH adjusted to pH 7 with 30% ammonia, were prepared as described in Example 1. These solutions were mixed in amounts to obtain a Cu-Fe-Ni solution of which 1.1 mL was impregnated on 1 g silica (Grace Davison, Davicat® SI 1351) to obtain a final catalyst composition of 10.3 %wt of total metal loading, with a Cu to Fe atomic ratio of 3:1, and 0.1 %at/at Ni relative to Cu and Fe. The impregnated support was homogenized and the catalyst precursor thus obtained was dried at 120 °C for 2 h and calcined at 450 °C for 2 h in air.

### Example 7 Preparation of a Cu-Zn on silica catalyst

Solutions (0.3 g salt/mL solution) of copper and zinc nitrates, with equimolar amounts of citric acid, and their pH adjusted to pH 7 with 30% ammonia, were prepared as described in Example 1. The solutions were mixed in amounts to obtain a Cu-Zn solution of which 2.5 mL was impregnated on 1 g silica (PQ, CS 2050) to obtain a final catalyst composition of 27.8 %wt of total metal loading, with a Cu to Zn atomic ratio of 3:1. The impregnated support was homogenized and the catalyst precursor thus obtained was dried at 120 °C for 2 h and calcined at 450 °C for 2 h in air. To obtain the 27.8 %wt metals loading, the support was twice impregnated, with a drying/calcination step in between.

### Example 8 Preparation of a Cu-Zn on silica catalyst

Copper citrate and zinc citrate solutions were prepared as in Example 2. To a sample of 1 g silica (Aerosil 300) was added 1.52 mL of the Cu solution, and 0.64 mL of the Zn solution, after which the slurry was stirred for five minutes. The slurry was then dried at 120 °C for 12 h and calcined at 450 °C for 2 h. The catalyst precursor was crushed to a powder. The catalyst composition was 20 g Cu-Zn per 100 g catalyst with a Cu to Zn at/at ratio of 3:1.

### Example 8A Preparation of a Cu-Zn-Rh on silica catalyst

A Cu-Zn catalyst precursor was prepared equivalent to Example 8. A third solution was prepared by dissolving 0.1 g of a rhodium nitrate solution (14.7% Rh, Chempur) in 10 mL water. The Cu-Zn on silica catalyst precursor was impregnated with a solution 0.292 mL of the Rh solution and sufficient additional water to reach incipient wetness. The wet powder was dried at 120 °C for 2 h and calcined at 450 °C for 2 h. The catalyst composition was 20 g Cu-Zn-Rh per 100 g catalyst with a Cu to Zn to Rh at/at ratio of 3:1:0.004.

### Example 8B Preparation of a Cu-Zn-Ru on silica catalyst

A catalyst was prepared analogous to Example 8A, except that the catalyst precursor powder was impregnated with a solution of ruthenium nitrosyl (13.0% Ru, Chempur). The catalyst composition was 20 g Cu-Zn-Ru per 100 g catalyst with a Cu to Zn to Ru at/at ratio of 3:1:0.004.

### Example 8C Preparation of a Cu-Zn-Pd on silica catalyst

A catalyst was prepared analogous to Example 8A, except that the catalyst precursor powder was impregnated with a solution of palladium nitrate (40.5% Pd, Chempur). The catalyst composition was 20 g Cu-Zn-Pd per 100 g catalyst with a Cu to Zn to Pd at/at ratio of 3:1:0.004.

### Example 8D Preparation of a Cu-Zn-Pt on silica catalyst

Solutions (0.5 g salt/mL solution) of copper and zinc nitrates, with equimolar amounts of citric acid, and their pH adjusted to pH 7 with 30% ammonia, were prepared as described in Example 1. To a sample of 1 g silica (Aerosil 300, Degussa) was added 3.77 mL of the Cu solution and 1.55 mL of the Zn solution, after which the slurry was stirred for five minutes. The slurry was then dried at 120 °C for 12 h and calcined at 450 °C for 2 h. The catalyst precursor was crushed to a powder. A third solution was prepared by dissolving 0.1 g of a platinum nitrate solution (58.2% Pt, Chempur) in 10 mL water. The Cu-Zn on silica catalyst precursor was impregnated with 0.139 mL Pt solution and sufficient additional water to reach incipient wetness. The wet powder was dried at 120 °C for 2 h and calcined at 450 °C for 2 h. The catalyst composition was 20 g Cu-Zn-Pt per 100 g catalyst with a Cu to Zn to Pt at/at ratio of 3:1:0.004.

### Example 9 Preparation of a Cu-Zn on zirconia catalyst

A catalyst was prepared analogous to Example 8. Instead of silica, zirconia powder was used (NNC100, Daiichi). The catalyst composition was 20 g Cu-Zn per 100 g catalyst with a Cu to Zn at/at ratio of 1:1.

### Example 9A Preparation of a Cu-Zn-Rh on zirconia catalyst

A catalyst was prepared analogous to Example 8A. Instead of silica, zirconia powder was used (NNC100, Daiichi). The catalyst composition was 20 g Cu-Zn-Rh per 100 g catalyst with a Cu to Zn to Rh at/at ratio of 1:1:0.004.

### Example 9B Preparation of a Cu-Zn-Ru on zirconia catalyst

A Cu-Zn catalyst precursor was prepared analogous to Example 8D. Instead of silica, zirconia powder was used (NNC100, Daiichi). The Ru was added analogous to Example 8B. The catalyst composition was 20 g Cu-Zn-Ru per 100 g catalyst with a Cu to Zn to Ru at/at ratio of 3:1:0.004.

### Example 9C Preparation of a Cu-Zn-Pd on zirconia catalyst

A catalyst was prepared analogous to Example 8C. Instead of silica, zirconia powder was used (NNC100, Daiichi). The catalyst composition was 20 g Cu-Zn-Pd per 100 g catalyst with a Cu to Zn to Pd at/at ratio of 1:1:0.004.

### Example 9D Preparation of a Cu-Zn-Pt on zirconia catalyst

A catalyst was prepared analogous to Example 8D. Instead of silica, zirconia powder was used (NNC100, Daiichi). The catalyst composition was 20 g Cu-Zn-Pt per 100 g catalyst with a Cu to Zn to Pt at/at ratio of 1:1:0.004.

### Example 10 Preparation of a Cu-Zn on magnesia catalyst

A Cu-Zn catalyst precursor was prepared analogous to Example 8D. Instead of silica, magnesia powder was used (E-10, DSP). The catalyst composition was 20 g Cu-Zn per 100 g catalyst with a Cu to Zn at/at ratio of 1:1.

### Example 10A Preparation of a Cu-Zn-Rh on magnesia catalyst

A Cu-Zn catalyst precursor was prepared analogous to Example 8D. Instead of silica, magnesia powder was used (E-10, DSP). The Rh was added analogous to Example 8A. The catalyst composition was 20 g Cu-Zn-Rh per 100 g catalyst with a Cu to Zn to Rh at/at ratio of 1:1:0.004.

### Example 10B Preparation of a Cu-Zn-Ru on magnesia catalyst

A catalyst was prepared analogous to Example 8B. Instead of silica, magnesia powder was used (E-10, DSP). The catalyst composition was 20 g Cu-Zn-Ru per 100 g catalyst with a Cu to Zn to Ru at/at ratio of 3:1:0.004.

### Example 10C Preparation of a Cu-Zn-Pd on magnesia catalyst

A catalyst was prepared analogous to Example 8C. Instead of silica, magnesia powder was used (E-10, DSP). The catalyst composition was 20 g Cu-Zn-Pd per 100 g catalyst with a Cu to Zn to Pd at/at ratio of 1:1:0.004.

### Example 10D Preparation of a Cu-Zn-Pt on magnesia catalyst

A catalyst was prepared analogous to Example 8D. Instead of silica, magnesia powder was used (E-10, DSP). The catalyst composition was 20 g Cu-Zn-Pt per 100 g catalyst with a Cu to Zn to Pt at/at ratio of 1:1:0.004.

### Examples 11 Preparation of Cu-Fe on silica catalyst

A catalyst was prepared analogous to Example 8A, using a Cu citrate solution (as in Example 2) and an iron citrate solution (as in Example 4). The catalyst compositions were 20 g Cu-Fe per 100 g catalyst with a Cu to Fe at/at ratio of 1:1.

### Examples 11A - 11D Preparation of Cu-Fe-(Rh, Ru, Pd, or Pt) on silica catalysts

Catalysts were prepared analogous to Example 8A, using a Cu citrate solution (as in Example 2) and an iron citrate solution (as in Example 4). The catalyst compositions were 20 g Cu-Fe-(Rh, Ru, Pd, or Pt) per 100 g catalyst with a Cu to Fe to (Rh, Ru, Pd, or Pt) at/at ratio of 1:1:0.004.

### Examples 12 Preparation of a Cu-Fe on silica catalyst

A catalyst was prepared analogous to Example 8D, using a Cu nitrate solution and an iron nitrate solution (as used in Example 6). The catalyst compositions were 20 g Cu-Fe per 100 g catalyst with a Cu to Fe at/at ratio of 1:1.

### Examples 12A - 12D Preparation of Cu-Fe-(Rh, Ru, Pd, or Pt) on silica catalysts

Catalysts were prepared analogous to Example 8D, using a Cu nitrate solution and an iron nitrate solution (as used in Example 6). The catalyst compositions were 20 g Cu-Fe-(Rh, Ru, Pd, or Pt) per 100 g catalyst with a Cu to Fe to (Rh, Ru, Pd, or Pt) at/at ratio of 1:1:0.004.

### Example 13 Catalyst testing procedure

A catalyst sample of 0.25 g (as prepared by the methods described in Examples 1-12) was reduced under a hydrogen flow for 2 h at a temperature of 350 °C. The reduced catalyst was transferred to a reactor and suspended in 25 mL methyl laurate. The reaction mixture was stirred at 750 rpm under 100 bar hydrogen and at 250 °C for 4 h. After cooling, the reaction mixture was analysed by GC. The results of the GC analysis are listed in Tables 1-3.

**Table 1 Test results of (promoted) Cu-(Fe or Zn) catalysts**

| CAT | CATALYST COMPOSITION | | | | | | | | | | | Selectivity | | | |
|---|---|---|---|---|---|---|---|---|---|---|---|---|---|---|---|
| | M1 | %wt | M2 | %wt | M3 | %wt | M4 | %wt | S | PREC | CONV | Lalc | LL | Lac | DD |
| | | | | | | | | | | | (%) | (%) | (%) | (%) | (%) |
| Ex.1 | Cu | 15 | Fe | 5 | | | | | sill | A | 55.3 | 83.9 | 15.8 | 0.0 | 0.2 |
| Ex.2A | Cu | 10 | Zn | 10 | | | | | mag | B | 41.6 | 44.9 | 51.9 | 3.0 | 0.0 |
| Ex.2B | Cu | 15 | Zn | 5 | Co | 0.2 | | | mag | B | 45.7 | 46.9 | 48.2 | 4.8 | 0.0 |
| Ex.2C | Cu | 15 | Zn | 5 | Co | 0.2 | | | mag | A | 60.8 | 59.8 | 37.4 | 2.6 | 0.0 |
| Ex.3 | Cu | 15 | Zn | 5 | | | | | zir | B | 45.7 | 64.3 | 25.8 | 9.8 | 0.0 |
| Ex.4 | Cu | 15 | Fe | 5 | Co | 0.2 | | | tit | B | 46.6 | 73.0 | 26.8 | 0.1 | 0.0 |
| Ex. 5A | Cu | 15 | Zn | 5 | Ni | 0.2 | | | mag | B | 39.4 | 35.7 | 61.9 | 2.2 | 0.0 |
| Ex. 5B | Cu | 15 | Zn | 5 | Ni | 0.2 | | | zir | B | 55.9 | 82.9 | 16.6 | 0.4 | 0.0 |
| Ex.6 | Cu | 8.0 | Fe | 2.3 | Ni | 0.01 | | | si12 | A | 35.3 | 82.3 | 17.2 | 0.3 | 0.0 |
| Ex.7 | Cu | 20.7 | Zn | 7.1 | | | | | sil3 | A | 57.7 | 72.0 | 27.9 | 0.0 | 0.0 |
| Comp. Ex. 1 | Cu | 15 | Fe | 5 | | | | | sill | C | 50.0 | 50.0 | 10.0 | 2.0 | 0.0 |
| Comp. Ex.2 | Cu | 38.5 | Cr | 28.3 | Ba | 4.6 | Mn | 3.5 | | | 18.0 | 80.8 | 17.4 | 1.8 | 0.0 |

| | | | | | | | | | | | | | | | |
|---|---|---|---|---|---|---|---|---|---|---|---|---|---|---|---|
| Test conditions: 250 °C, 100 bar hydrogen, 4 h, 750 rpm, 25 mL methyl laurate feed Metal precursor (PREC): A = metal nitrate + equimolar citric acid + ammonia (pH 7); B = metal citrate in 30% ammonia; C = metal nitrate. Abbreviations used: CAT = catalyst; M1 = metal 1, etc.; S = support; CONV = percent conversion of lauric methyl ester; Lalc = lauryl alcohol; LL = lauryl laurate; Lac = lauric acid; DD = dodecane Supports: sill = Aerosil 300 (Degussa); sil2 = Davicat® SI 1351 (Grace Davison); sil3 = CS 2050 (PQ) mag = E-10 (DSP); zir = NNC100 (Daiichi); tit = P25 (Degussa) | | | | | | | | | | | | | | | |

**Table 2 Test results of unpromoted and noble metal promoted Cu-Zn catalysts**

| | | | | | | | | | | | | | |
|---|---|---|---|---|---|---|---|---|---|---|---|---|---|
| CAT | CATALYST COMPOSITION | | | | | | | | | Selectivity | | | |
| | M1 | %wt | M2 | %wt | M3 | %wt | S | PREC | CONV | Lalc | LL | Lac | DD |
| | | | | | | | | | (%) | (%) | (%) | (%) | (%) |
| Ex. 8 | Cu | 15 | Zn | 5 | | | sill | B | 69.9 | 83.7 | 16.1 | 0.1 | 0.0 |
| Ex.8A | Cu | 15 | Zn | 5 | Rh | 0.03 | sill | B | 57.8 | 79.9 | 19.8 | 0.1 | 0.0 |
| Ex. 8B | Cu | 15 | Zn | 5 | Ru | 0.03 | sill | B | 55.5 | 82.3 | 17.2 | 0.3 | 0.0 |
| Ex.8C | Cu | 15 | Zn | 5 | Pd | 0.03 | sill | B | 46.6 | 82.6 | 17.1 | 0.2 | 0.0 |
| Ex.8D | Cu | 15 | Zn | 5 | Pt | 0.06 | sill | A | 50.5 | 76.8 | 23.1 | 0.0 | 0.0 |
| Ex. 9 | Cu | 10 | Zn | 10 | | | zir | B | 38.1 | 72.9 | 25.8 | 1.1 | 0.0 |
| Ex.9A | Cu | 10 | Zn | 10 | Rh | 0.03 | zir | B | 55.5 | 78.8 | 20.5 | 0.5 | 0.0 |
| Ex.9B | Cu | 15 | Zn | 5 | Ru | 0.03 | zir | A | 53.0 | 79.9 | 19.7 | 0.3 | 0.0 |
| Ex.9C | Cu | 10 | Zn | 10 | Pd | 0.03 | zir | B | 47.4 | 81.3 | 17.0 | 1.6 | 0.0 |
| Ex.9D | Cu | 10 | Zn | 10 | Pt | 0.06 | zir | A | 55.6 | 83.7 | 15.8 | 0.4 | 0.0 |
| Ex.10 | Cu | 10 | Zn | 10 | | | mag | A | 48.7 | 58.1 | 39.8 | 1.9 | 0.0 |
| Ex.10A | Cu | 10 | Zn | 10 | Rh | 0.03 | mag | A | 47.3 | 48.3 | 50.4 | 1.1 | 0.0 |
| Ex.10B | Cu | 15 | Zn | 5 | Ru | 0.03 | mag | B | 41.1 | 44.0 | 53.2 | 2.6 | 0.0 |
| Ex.10C | Cu | 15 | Zn | 5 | Pd | 0.03 | mag | B | 43.9 | 44.4 | 50.8 | 4.6 | 0.0 |
| Ex.10D | Cu | 10 | Zn | 10 | Pt | 0.06 | mag | A | 44.5 | 50.4 | 48.1 | 1.4 | 0.0 |

| | | | | | | | | | | | | | |
|---|---|---|---|---|---|---|---|---|---|---|---|---|---|
| See Table 1 for abbreviations and test conditions. | | | | | | | | | | | | | |

**Table 3 Test results of unpromoted and noble metal promoted Cu-Fe catalysts**

| CAT | CATALYST COMPOSITION | | | | | | | | | Selectivity | | | |
|---|---|---|---|---|---|---|---|---|---|---|---|---|---|
| | M1 | %wt | M2 | %wt | M3 | %wt | S | PREC | CONV | Lalc | LL | Lac | DD |
| | | | | | | | | | (%) | (%) | (%) | (%) | (%) |
| Ex.11 | Cu | 10 | Fe | 10 | | | sill | B | 37.9 | 74.8 | 24.9 | 0.1 | 0.0 |
| Ex.11A | Cu | 10 | Fe | 10 | Rh | 0.03 | sill | B | 38.6 | 66.3 | 33.1 | 0.0 | 0.0 |
| Ex.11B | Cu | 10 | Fe | 10 | Ru | 0.03 | sill | B | 39.8 | 77.7 | 22.0 | 0.2 | 0.0 |
| Ex.11C | Cu | 10 | Fe | 10 | Pd | 0.03 | sill | B | 38.9 | 76.0 | 23.5 | 0.4 | 0.0 |
| Ex.11D | Cu | 10 | Fe | 10 | Pt | 0.06 | sill | B | 37.0 | 66.3 | 33.1 | 0.5 | 0.0 |
| Ex.12 | Cu | 10 | Fe | 10 | | | sill | A | 56.4 | 79.6 | 20.3 | 0.0 | 0.0 |
| Ex.12A | Cu | 10 | Fe | 10 | Rh | 0.03 | sill | A | 55.4 | 80.9 | 19.0 | 0.0 | 0.0 |
| Ex.12B | Cu | 10 | Fe | 10 | Ru | 0.03 | sill | A | 52.1 | 78.5 | 21.0 | 0.3 | 0.0 |
| Ex. 12C | Cu | 10 | Fe | 10 | Pd | 0.03 | sill | A | 48.2 | 80.3 | 19.6 | 0.0 | 0.0 |
| Ex.12D | Cu | 10 | Fe | 10 | Pt | 0.06 | sill | A | 51.9 | 80.5 | 19.4 | 0.0 | 0.0 |

| | | | | | | | | | | | | | |
|---|---|---|---|---|---|---|---|---|---|---|---|---|---|
| See Table 1 for abbreviations and test conditions. | | | | | | | | | | | | | |

## Claims

1. Method for the preparation of a chromium-free catalyst comprising Cu and at least one second metal in metallic or oxidic form, comprising the steps of:
a) preparing a final solution comprising ions of Cu and of at least one second metal, said final solution additionally comprising ions of an organic complexing agent and having a pH of above 5;
b) contacting said final solution with inert carrier to form a final solution/carrier combination;
c) optionally, drying the final solution/carrier combination;
d) calcining the final solution/carrier combination obtained in step c) or d) to yield Cu and the at least one second metal in oxidic form; and
e) reducing at least part of the thus obtained oxidic Cu on the carrier.

2. Method according to claim 1, wherein the organic complexing agent is chosen from citric acid, lactic acid, and EDTA.

3. Method according to any of claims1 or 2, step a) comprising the step of preparing said final solution by combining at least a first solution comprising ions of Cu with at least a second solution comprising ions of at least one second metal.

4. Method according to claim 3, wherein the first and second solutions both comprise ions of the complexing agent in a similar concentration.

5. Method according to any of claims 3 or 4, wherein both the first solution and the second solution have a pH of above 5.

6. Method according to claim 5, wherein the first and the second solution.have a similar pH.

7. Method according to any of the preceding claims, wherein said chromium-free catalyst further comprises at least one third metal.

8. Method according to any of the preceding claims, wherein the pH of the final solution is above 6.

9. Method according to any of the preceding claims, wherein the concentration of Cu ions in the final solution is in the range of 0.001-0.3, more preferably of 0.005-0.15 g Cu/mL.

10. Method according to any of the preceding claims, wherein the amount of Cu ions in the final solution is such that a catalyst is obtained comprising 1-50 %wt, more preferably 10 to 30 %wt, and most preferably 15 - 25 %wt Cu.

11. Method according to any of the preceding claims, wherein the concentration of ions of the complexing agent in the final solution is in the range of 0.001-1.5, more preferably of 0.15-0.5 g/mL.

12. Method according to any of the preceding claims, wherein the amount of ions of the complexing agent in the final solution is such that the molar ratio of metal to complexing agent is in the range of 0.1 to 5, more preferably 0.5 to 2, and most preferably 0.75-1.25.

13. Method according to any of the preceding claims, wherein the concentration of ions of the at least one second metal in the final solution is in the range of 0.001-0.3, preferably in the range of 0.005-0.15 g/mL.

14. Method according to any of the preceding claims, wherein the amount of ions of the at least one second metal in the final solution is such that catalyst is obtained with an atomic ratio of Cu to the at least one second metal in the range of 0.01-10, more preferably in the range of 0.1-5, and most preferably in the range of 0.3-3.0.

15. Method according to any of the claims 7-14, wherein the concentration of ions of the at least one third metal in the final solution is in the range of 0.0001-0.03, preferably in the range of 0.0005-0.015 g/mL.

16. Method according to any of the claims 7-15, wherein the amount of the at least one third metal is such that catalyst is obtained with an atomic ratio of the at least one third metal to Cu in the range of 0.001-0.05, more preferably in the range of 0.001-0.01.

17. Method according to any of the preceding claims, comprising an additional step g) of pulverising the obtained catalyst.

18. Method according to any of the preceding claims, wherein the at least one second metal is chosen from one or more of Fe, Zn, Co, Ni; or a combination thereof.

19. Method according to any of the preceding claims, wherein the at least one third metal is chosen from one or more of Pd, Ru, Pt, Rh, or a combination of two or more thereof.

20. Method according to any of the preceding claims, wherein the inert carrier is chosen from alumina, silica, silica-alumina, titania, magnesia, zirconia, zinc oxide, or any combination thereof.

21. Method according to any of the preceding claims, wherein the inert carrier is present in an amount of 50-95 %wt.

22. Chromium-free catalyst comprising Cu and at least one second metal obtainable by a method according to any of the preceding claims.

23. Chromium-free catalyst according to claim 22, said catalyst comprising at least 5 %wt Cu and having an atomic ratio of Cu to the at least one second metal of 0.1-10.

24. Chromium-free Cu-Zn catalyst supported on silica, zirconia, or magnesia according to any of claims 22 or 23, comprising 5-50 %wt, preferably 10-30 %wt (Cu + Zn) and having a Cu to Zn ratio of 0.1-10 at/at, preferably 0.5-5 at/at, more preferably 1-4 at/at.

25. Chromium-free Cu-Zn catalyst according to claim 24, further comprising as at least one second metal Co or Ni, or a combination thereof.

26. Chromium-free Cu-Zn catalyst according to any of claims 24 or 25, further comprising at least one third metal chosen from Rh, Ru, Pd and Pt, or combinations of two or more thereof.

27. Chromium-free Cu-Zn catalyst according to claim 26 having a ratio of (Cu + Zn) to the at least one third metal of 0.0001-0.5 at/at, preferably of 0.001-0,01 at/at.

28. Chromium-free Cu-Fe catalyst,supported on silica, zirconia, or magnesia according to any of claims 22 or 23, comprising 5-50 %wt, preferably 10-30 %wt (Cu + Fe) and having a Cu to Fe ratio of 0.1-10 at/at, preferably 0.5-5 at/at, more preferably 1-4 at/at.

29. Chromium-free Cu-Fe catalyst according to claim 28, further comprising as at least one second metal Co or Ni, or a combination thereof.

30. Chromium-free Cu-Fe catalyst according to any of claims 28 or 29, further comprising at least one third metal chosen from Rh, Ru, Pd and Pt, or combinations of two or more thereof.

31. Chromium-free Cu-Fe catalyst according to claim 30 having a ratio of (Cu + Fe) to the at least one third metal of 0.0001-0.5 at/at, preferably of 0.001-0.01 at/at.

32. Use of chromium-free catalyst according to any of claims 22-31 for the hydrogenation of fatty acids, fatty esters, esters and diesters to fatty alcohols, alcohols and dialcohols, respectively.

## Patentansprüche

1. Verfahren zur Herstellung eines Chrom-freien Katalysators, der Cu und mindestens ein zweites Metall in metallischer oder Oxidform enthält, enthaltend die Schritte:
a) Herstellen einer Fertiglösung, die Ionen von Cu und mindestens einem zweiten Metall enthält, welche Fertiglösung zusätzlich Ionen eines organischen Komplexbildners enthält und einen pH über 5 hat;
b) Kontaktieren der Fertiglösung mit einem inerten Träger, um eine Fertiglösung-/Trägerkombination zu bilden;
c) optional Trocknen der Fertiglösung-/Trägerkombination;
d) Calcinieren der in Schritt c) oder d) erhaltenen Fertiglösung-/Trägerkombination, um Cu und das mindestens eine zweite Metall in Oxidform zu erhalten; und
e) Reduzieren mindestens eines Teiles des auf diese Weise erhaltenen oxidischen Cu an dem Träger.

2. Verfahren nach Anspruch 1, bei welchem der organische Komplexbildner ausgewählt wird aus Zitronensäure, Milchsäure und EDTA.

3. Verfahren nach einem der Ansprüche 1 oder 2, wobei Schritt a) den Schritt des Herstellens der Fertiglösung durch Kombinieren mindestens einer ersten Lösung, die Cu-Ionen enthält, mit mindestens einer zweiten Lösung enthält, die Ionen mindestens eines zweiten Metalls enthält.

4. Verfahren nach Anspruch 3, bei welchem sowohl die erste als auch die zweite Lösung Ionen des Komplexbildners in einer ähnlichen Konzentration enthalten.

5. Verfahren nach einem der Ansprüche 3 oder 4, bei welchem sowohl die erste Lösung als auch die zweite Lösung einen pH über 5 haben.

6. Verfahren nach Anspruch 5, bei welchem die erste und die zweite Lösung einen ähnlichen pH haben.

7. Verfahren nach einem der vorstehenden Ansprüche, bei welchem der Chrom-freie Katalysator ferner mindestens ein drittes Metall enthält.

8. Verfahren nach einem der vorstehenden Ansprüche, bei welchem der pH der Fertiglösung über 6 ist.

9. Verfahren nach einem der vorstehenden Ansprüche, bei welchem die Konzentration von Cu-Ionen in der Fertiglösung im Bereich von 0,001-0,3 , bevorzugter von 0,005-0,15 g Cu/ml ist.

10. Verfahren nach einem der vorstehenden Ansprüche, bei welchem die Menge von Cu-Ionen in der Fertiglösung dergestalt ist, dass ein Katalysator erhalten wird, der 1-50 Gew.-%, bevorzugter 10 - 30 Gew.-% und höchst bevorzugt 15-25 Gew.-% Cu enthält.

11. Verfahren nach einem der vorstehenden Ansprüche, bei welchem die Konzentration der Ionen des Komplexbildners in der Fertiglösung im Bereich von 0,001-1,5 , bevorzugter von 0,15-0,5 g/ml ist.

12. Verfahren nach einem der vorstehenden Ansprüche, bei welchem die Menge der Ionen des Komplexbildners in der Fertiglösung dergestalt ist, dass das Molverhältnis von Metall zu Komplexbildner im Bereich von 0,1 bis 5, bevorzugter 0,5 bis 2 und höchst bevorzugt 0,75 bis 1,25 liegt.

13. Verfahren nach einem der vorstehenden Ansprüche, bei welchem die Konzentration von Ionen des mindestens einen zweiten Metalls in der Fertiglösung im Bereich von 0,001-0,3 , vorzugsweise im Bereich von 0,005-0,15 g/ml ist.

14. Verfahren nach einem der vorstehenden Ansprüche, bei welchem die Menge von Ionen des mindestens einen zweiten Metalls in der Fertiglösung dergestalt ist, dass ein Katalysator mit einem Atomverhältnis von Cu zu dem mindestens einen zweiten Metall im Bereich von 0,01-10, bevorzugter im Bereich von 0,1-5 und höchst bevorzugt im Bereich von 0,3-3,0 erhalten wird.

15. Verfahren nach einem der Ansprüche 7-14, bei welchem die Konzentration von Ionen des mindestens einen dritten Metalls in der Fertiglösung im Bereich von 0,0001-0,03 , bevorzugt im Bereich von 0,0005-0,015 g/ml liegt.

16. Verfahren nach einem der Ansprüche 7-15, bei welchem die Menge des mindestens einen dritten Metalls dergestalt ist, dass ein Katalysator mit einem Atomverhältnis des mindestens einen dritten Metalls zu Cu im Bereich von 0,001-0,05 , bevorzugter im Bereich von 0,001-0,01 erhalten wird.

17. Verfahren nach einem der vorstehenden Ansprüche, enthaltend einen zusätzlichen Schritt g) des Pulverisierens des erhaltenen Katalysators.

18. Verfahren nach einem der vorstehenden Ansprüche, bei welchem das mindestens eine zweite Metall ausgewählt ist aus einem oder mehreren von Fe, Zn, Co, Ni oder einer Kombination daraus.

19. Verfahren nach einem der vorstehenden Ansprüche, bei welchem das mindestens eine dritte Metall ausgewählt ist aus einem oder mehreren von Pd, Ru, Pt, Rh oder einer Kombination von zwei oder mehreren daraus.

20. Verfahren nach einem der vorstehenden Ansprüche, bei welchem der inerte Träger ausgewählt ist aus Aluminiumoxid, Siliciumoxid, Silicium-Aluminiumoxid, Titanoxid, Magnesiumoxid, Zirconiumoxid, Zinkoxid oder einen beliebigen Kombination daraus.

21. Verfahren nach einem der vorstehenden Ansprüche, bei welchem der inerte Träger in einer Menge von 50-95 Gew.-% vorhanden ist.

22. Chrom-freier Katalysator, enthaltend Cu und mindestens ein zweites Metall, erzielbar durch ein Verfahren gemäß einem der vorstehenden Ansprüche.

23. Chrom-freier Katalysator nach Anspruch 22, welcher Katalysator mindestens 5 Gew.-% Cu enthält und ein Atomverhältnis von Cu zu dem mindestens einen zweiten Metall von 0,1-10 hat.

24. Chrom-freier Cu-Zn-Katalysator auf einem Siliciumoxid-, Zirconiumoxid- oder Magnesiumoxid-Träger gemäß einem der Ansprüche 22 oder 23, enthaltend 5-50 Gew.-%, vorzugsweise 10-30 Gew.-% (Cu + Zn) und mit einem Verhältnis von Cu zu Zn von 0,1-10 at/at, bevorzugt 0,5-5 at/at, bevorzugter 1-4 at/at.

25. Chrom-freier Cu-Zn-Katalysator nach Anspruch 24, ferner enthaltend als mindestens ein zweites Metall Co oder Ni oder eine Kombination daraus.

26. Chrom-freier Cu-Zn-Katalysator nach einem der Ansprüche 24 oder 25, ferner enthaltend mindestens ein drittes Metall, ausgewählt aus Rh, Ru, Pd und Pt, oder Kombinationen von zwei oder mehr daraus.

27. Chrom-freier Cu-Zn-Katalysator nach Anspruch 26 mit einem Verhältnis von (Cu + Zn) zu dem mindestens einen dritten Metall von 0,0001-0,5 at/at, bevorzugt von 0,001-0,01 at/at.

28. Chrom-freier Cu-Fe-Katalysator auf einem Siliciumoxid-, Zirconiumoxid- oder Magnesiumoxid-Träger gemäß einem der Ansprüche 22 oder 23, enthaltend 5-50 Gew.-%, vorzugsweise 10-30 Gew.-% (Cu + Fe) und mit einem Verhältnis von Cu zu Fe von 0,1-10 at/at, bevorzugt 0,5-5 at/at, bevorzugter 1-4 at/at.

29. Chrom-freier Cu-Fe-Katalysator nach Anspruch 28, ferner enthaltend als mindestens ein zweites Metall Co oder Ni oder eine Kombination daraus.

30. Chrom-freier Cu-Fe-Katalysator nach einem der Ansprüche 28 oder 29, ferner enthaltend mindestens ein drittes Metall, ausgewählt aus Rh, Ru, Pd und Pt, oder Kombinationen von zwei oder mehr daraus.

31. Chrom-freier Cu-Fe-Katalysator nach Anspruch 30 mit einem Verhältnis von (Cu + Fe) zu dem mindestens einen dritten Metall von 0,0001-0,5 at/at, bevorzugt von 0,001-0,01 at/at.

32. Verwendung eines Chrom-freien Katalysators gemäß einem der Ansprüche 22-31 für die Hydrierung von Fettsäuren, Fettsäureestern, Estern und Diestern zu Fettalkoholen, Alkoholen beziehungsweise Dialkoholen.

## Revendications

1. Procédé de préparation d'un catalyseur exempt de chrome comprenant du Cu et au moins un deuxième métal sous forme métallique ou oxydée, comprenant les stades dans lesquels:
a) on prépare une solution finale comprenant des ions de Cu et d'au moins un deuxième métal, la solution finale comprenant, en outre, des ions d'un agent organique complexant et ayant un pH supérieur à 5 ;
b) on met la solution finale en contact avec du support inerte pour former une combinaison solution finale/support ;
c) éventuellement on sèche la combinaison solution finale/support ;
d) on calcine la combinaison solution finale/support obtenue au stade c) ou d) pour obtenir du Cu et le au moins un deuxième métal sous forme oxydée ; et
e) on réduit au moins une partie du Cu oxydé ainsi obtenu sur le support.

2. Procédé suivant la revendication 1, dans lequel l'agent complexant organique est choisi parmi l'acide citrique, l'acide lactique et l'EDTA.

3. Procédé suivant l'une quelconque des revendications 1 ou 2, le stade a) comprenant le stade de préparation de la solution finale en combinant au moins une première solution comprenant des ions de Cu à au moins une deuxième solution comprenant des ions d'au moins un deuxième métal.

4. Procédé suivant la revendication 3, dans lequel les première et deuxième solutions comprennent toutes deux des ions de l'agent complexant en une concentration semblable.

5. Procédé suivant l'une quelconque des revendications 3 ou 4, dans lequel la première solution et la deuxième solution ont toutes deux un pH supérieur à 5.

6. Procédé suivant la revendication 5, dans lequel les première et deuxième solutions ont un pH semblable.

7. Procédé suivant l'une quelconque des revendications précédentes, dans lequel le catalyseur exempt de chrome comprend, en outre, au moins un troisième métal.

8. Procédé suivant l'une quelconque des revendications précédentes, dans lequel le pH de la solution finale est supérieur à 6.

9. Procédé suivant l'une quelconque des revendications précédentes, dans lequel la concentration en ions Cu de la solution finale est comprise entre 0,001 et 0,3 et, de préférence, entre 0,005 et 0,15 g de Cu/ml.

10. Procédé suivant l'une quelconque des revendications précédentes, dans lequel la quantité d'ions Cu dans la solution finale est telle que l'on obtient un catalyseur comprenant de 1 à 50 % en poids et, de préférence, de 10 à 30 % en poids et mieux encore de 15 à 25 % en poids de Cu.

11. Procédé suivant l'une quelconque des revendications précédentes, dans lequel la concentration en ions de l'agent complexant dans la solution finale est comprise entre 0,001 et 1,5 et, de préférence, entre 0,15 et 0,5 g/ml.

12. Procédé suivant l'une quelconque des revendications précédentes, dans lequel la quantité d'ions de l'agent complexant dans la solution finale est telle que le rapport molaire du métal à l'agent complexant est compris entre 0,1 et 5 et, de préférence, entre 0,5 et 2 et mieux encore entre 0,75 et 1,25.

13. Procédé suivant l'une quelconque des revendications précédentes, dans lequel la concentration en ions du au moins un deuxième métal dans la solution finale est comprise entre 0,001 et 0,3 et, de préférence, entre 0,005 et 0,15 g/ml.

14. Procédé suivant l'une quelconque des revendications précédentes, dans lequel la quantité d'ions du au moins un deuxième métal dans la solution finale est telle que l'on obtient du catalyseur ayant un rapport atomique de Cu au au moins un deuxième métal compris entre 0,01 et 10, de préférence entre 0,1 et 5 et mieux encore entre 0,3 et 3,0.

15. Procédé suivant l'une quelconque des revendications 7 à 14, dans lequel la concentration en ions du au moins un troisième métal dans la solution finale est comprise entre 0,0001 et 0,03, de préférence entre 0,0005 et 0,015 g/ml.

16. Procédé suivant l'une quelconque des revendications 7 à 15, dans lequel la quantité du au moins un troisième métal est telle que l'on obtient du catalyseur ayant un rapport atomique du au moins un troisième métal au Cu compris entre 0,001 et 0,05, et, de préférence, entre 0,001 et 0,01.

17. Procédé suivant l'une quelconque des revendications précédentes, comprenant un stade g) supplémentaire de réduction en poudre du catalyseur obtenu.

18. Procédé suivant l'une quelconque des revendications précédentes, dans lequel le au moins un deuxième métal est choisi parmi l'un ou plusieurs de Fe, Zn, Co, Ni ou leurs combinaisons.

19. Procédé suivant l'une quelconque des revendications précédentes, dans lequel le au moins un troisième métal est choisi parmi l'un ou plusieurs de Pd, Ru, Pt, Rh ou l'une de leurs combinaisons de deux ou plusieurs.

20. Procédé suivant l'une quelconque des revendications précédentes, dans lequel le support inerte est choisi parmi l'alumine, la silice, la silice-alumine, l'oxyde de titane, la magnésie, l'oxyde de zirconium, l'oxyde de zinc ou l'une quelconque de leurs combinaisons.

21. Procédé suivant l'une quelconque des revendications précédentes, dans lequel le support inerte est présent en une quantité de 50 à 95 % en poids.

22. Catalyseur exempt de chrome comprenant du Cu et au moins un deuxième métal qui peut être obtenu par un procédé suivant l'une quelconque des revendications précédentes.

23. Catalyseur exempt de chrome suivant la revendication 22, le catalyseur comprenant au moins 5 % en poids de Cu et ayant un rapport atomique de Cu au au moins un deuxième métal de 0,1 à 10.

24. Catalyseur au Cu-Zn exempt de chrome supporté sur silice, oxyde de zirconium ou magnésie suivant l'une quelconque des revendications 22 ou 23, comprenant de 5 à 50 % en poids, de préférence de 10 à 30 % en poids de (Cu + Zn) et ayant un rapport de Cu à Zn de 0,1 à 10 at/at, de préférence de 0,5 à 5 at/at et mieux encore de 1 à 4 at/at.

25. Catalyseur au Cu-Zn exempt de chrome suivant la revendication 24, comprenant, en outre, comme au moins un deuxième métal du Co ou du Ni ou l'une de leurs combinaisons.

26. Catalyseur au Cu-Zn exempt de chrome suivant l'une quelconque des revendications 24 ou 25, comprenant, en outre, au moins un troisième métal choisi parmi Rh, Ru, Pd et Pt ou leurs combinaisons de deux ou plusieurs.

27. Catalyseur au Cu-Zn exempt de chrome suivant la revendication 26 ayant un rapport de (Cu + Zn) au au moins un troisième métal de 0,0001 à 0,5 at/at, de préférence de 0,001 à 0,01 at/at.

28. Catalyseur au Cu-Fe exempt de chrome supporté sur silice, sur oxyde de zirconium ou sur magnésie suivant l'une quelconque des revendications 22 ou 23, comprenant de 5 à 50 % en poids, de préférence de 10 à 30 % en poids de (Cu + Fe) et ayant un rapport de Cu à Fe de 0,1 à 10 at/at, de préférence de 0,5 à 5 at/at et mieux encore de 1 à 4 at/at.

29. Catalyseur au Cu-Fe exempt de chrome suivant la revendication 28, comprenant, en outre, comme au moins un deuxième métal Co ou Ni ou l'une de leurs combinaisons.

30. Catalyseur au Cu-Fe exempt de chrome suivant l'une quelconque des revendications 28 ou 29, comprenant, en outre, au moins un troisième métal choisi parmi Rh, Ru, Pd et Pt ou leurs combinaisons de deux ou plusieurs.

31. Catalyseur au Cu-Fe exempt de chrome suivant la revendication 30 ayant un rapport de (Cu + Fe) au au moins un troisième métal de 0,0001 à 0,5 at/at, de préférence de 0,001 à 0,01 at/at.

32. Utilisation d'un catalyseur exempt de chrome suivant l'une quelconque des revendications 22 à 31 pour l'hydrogénation d'acides gras, d'esters gras, d'esters et de diesters en des alcools gras, en des alcools et en des dialcools, respectivement.
